(19) **Europäisches Patentamt European Patent Office Office européen des brevets**

(11) **EP 2 736 356 B1**

(12) **FASCICULE DE BREVET EUROPEEN**

(45) Date de publication et mention de la délivrance du brevet:
**12.04.2017 Bulletin 2017/15**

(21) Numéro de dépôt: **12758527.1**

(22) Date de dépôt: **30.07.2012**

(51) Int Cl.:
*A61K 8/92* (2006.01)  *A61Q 19/08* (2006.01)
*A61K 19/00* (2006.01)  *A61Q 19/06* (2006.01)
*A61K 8/97* (2017.01)  *A61K 8/99* (2017.01)
*C11B 1/04* (2006.01)  *C11B 1/10* (2006.01)
*A23L 33/105* (2016.01)  *A23L 33/11* (2016.01)
*A23L 33/15* (2016.01)  *C11B 13/00* (2006.01)

(86) Numéro de dépôt international:
**PCT/FR2012/000320**

(87) Numéro de publication internationale:
**WO 2013/014344 (31.01.2013 Gazette 2013/05)**

(54) **EXTRACTION LIQUIDE/LIQUIDE AVEC UN SOLVANT COMPRENANT AU MOINS 5 ATOMES DE CARBONE ET 1 OU 2 ATOMES D'OXYGENE**

FLÜSSIG/FLÜSSIG-EXTRAKTION MIT EINEM LÖSUNGSMITTEL MIT MINDESTENS 5 KOHLENSTOFFATOMEN UND 1 ODER 2 SAUERSTOFFATOMEN

LIQUID/LIQUID EXTRACTION WITH A SOLVENT COMPRISING AT LEAST 5 CARBON ATOMS AND 1 OR 2 OXYGEN ATOMS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.07.2011 FR 1156936**

(43) Date de publication de la demande:
**04.06.2014 Bulletin 2014/23**

(73) Titulaire: **Laboratoires Expanscience 92400 Courbevoie (FR)**

(72) Inventeurs:
• **MERCIER, Eglantine**
  **F-78120 Rambouillet (FR)**
• **LEGRAND, Jacques**
  **F-61290 Neuilly sur Eure (FR)**
• **SAUNOIS, Alex**
  **F-28210 Nogent-le-Roi (FR)**

(74) Mandataire: **Regimbeau**
  **20, rue de Chazelles**
  **75847 Paris Cedex 17 (FR)**

(56) Documents cités:
**EP-A1- 1 733 731     FR-A1- 2 951 736**
**US-A- 2 530 809     US-A- 3 804 819**

• **DATABASE WPI Week 200915 Thomson Scientific, London, GB; AN 2009-B34086 XP002669763, "Extraction of sterol from raw glycerol comprises adding alkaline ethanol aqueous solution into extracted glycerol, heating and saponifying, adding extraction agent, stirring, leaving or centrifuging, washing, extracting, and drying", & CN 101 318 988 A (UNIV HUAZHONG AGRIC) 10 décembre 2008 (2008-12-10)**

Remarques:
Le dossier contient des informations techniques présentées postérieurement au dépôt de la demande et ne figurant pas dans le présent fascicule.

EP 2 736 356 B1

**Description**

La présente invention concerne un procédé d'extraction de fractions insaponifiables, notamment partielles ou totales, à partir d'huiles ou de beurres végétaux ou d'huiles provenant de micro-organismes.

Les insaponifiables ou fractions insaponifiables d'un corps gras sont constitués de composés qui, après action prolongée d'une base alcaline, restent insolubles dans l'eau et peuvent être extraits par un solvant organique.

La plupart des insaponifiables d'huiles ou de beurres végétaux comprennent plusieurs grandes familles de substances. Parmi ces grandes familles, on peut citer les hydrocarbures saturés ou insaturés, les alcools aliphatiques ou terpéniques, les stérols, les tocophérols, les pigments caroténoïdes, xanthophiles, ainsi que des familles spécifiques, notamment une ou deux, dans le cas de certaines huiles et beurres.

Les procédés usuels d'obtention des insaponifiables des huiles végétales et des beurres végétaux visent à extraire tout ou partie des grandes familles qui les composent, permettant de préparer les fractions partielles ou totales des insaponifiables.

Les fractions partielles ou totales d'insaponifiables sont notamment recherchées pour leurs propriétés pharmacologiques, cosmétiques et nutritionnelles.

Les procédés usuels d'obtention des insaponifiables des huiles végétales et des beurres végétaux comprennent une étape de saponification de la matière grasse et une extraction du produit cible (l'insaponifiable) par un solvant organique. Des procédés d'extractions de stérols sont notamment décrits dans les documents US 2 530 809 et CN 101 318 988.

Si divers solvants d'extraction peuvent être utilisés (voir FR 2 951 736, US 3 804 819 et EP 1 733 731 pour les extractions liquide-liquide), les solvants les plus communément utilisés sont les solvants des huiles, tels que les alcanes (hexane, heptane,...) et les solvants chlorés (1,2-dichloroéthane ou DCE, trichloroéthane, 1-chlorobutane, tétrachlorure de carbone,...). Parmi ces derniers, le DCE et le 1-chlorobutane constituent les meilleurs candidats, notamment au regard de leur rendement d'extraction et de leur sélectivité.

Cependant, sur le plan industriel, la toxicité du solvant mis en oeuvre, ainsi que sa stabilité chimique, doivent être pris en compte.

Les solvants chlorés présentent fréquemment une toxicité, une écotoxicité et/ou une dangerosité indésirable(s).

A ce titre, les solvants chlorés, et en particulier le 1,2-dichloroéthane (DCE) et le 1-chlorobutane, présentent trois inconvénients majeurs : ils peuvent se dégrader en milieu basique (ce qui est le cas des solutions savonneuses de saponification), ils sont classés parmi les solvants toxiques, notamment CMR pour le DCE, et ils provoquent des impacts négatifs sur l'environnement (écotoxicité).

Par ailleurs, tant d'un point de vue économique que d'un point de vue environnemental, les procédés d'obtention de fractions insaponifiables peuvent requérir l'utilisation de quantités de solvants organiques non adaptées pour la viabilité du procédé, présenter un nombre d'étapes d'extraction insatisfaisant, être trop lents et/ou présenter des séparations de phase insatisfaisantes, par exemple en provoquant une émulsion non désirée.

La présente invention vise donc à résoudre en tout ou partie les problèmes évoqués ci-dessus. En particulier, l'invention vise à fournir un procédé présentant un rendement global plus élevé, un taux d'extraction amélioré en une ou plusieurs fractions d'insaponifiable, plus économique, plus direct, plus amical pour l'environnement, nécessitant une quantité de solvant organique plus faible, plus facile à mettre en oeuvre, plus rapide, générant des conditions moins toxiques et/ou moins dangereuses, améliorant la séparation de phases, permettant l'obtention d'insaponifiables avec un rendement, un coût et/ou une sélectivité au moins comparable(s) aux procédés déjà existants.

En particulier, il est souhaitable que le(s) solvant(s) impliqué(s) soi(en)t moins dangereux, moins toxique(s), notamment non-classé(s) CMR, soi(en)t chimiquement plus stable(s) que le 1,2-dichloroéthane et/ou que le 1-chlorobutane et/ou permette(nt) d'extraire les insaponifiables avec un rendement et/ou une sélectivité au moins comparables aux rendements et sélectivités obtenus en utilisant le 1,2-dichloroéthane et/ou le 1-chlorobutane. Les solvants dits « classés CMR » peuvent être ceux qui sont présentés dans la liste en annexes de la directive 2009/2/CE du 15 janvier 2009, cette première liste étant appelée ci-après « liste CMR UE1 », ceux listés dans la Classification européenne réglementaire des produits chimiques cancérogènes, mutagènes et toxiques pour la reproduction - 31e ATP, 2009, cette deuxième liste étant appelée ci-après « liste CMR UE2 », et/ou ceux listés dans la liste « Chemicals known or suspected to cause cancer or reproductive toxicity » du 1er septembre 2009 établie par le « California department of public health, occupational health branch, California safe cosmetic program » liée à la « California Safe Cosmetics Act of 2005 », cette troisième liste étant appelée ci-après « liste CMR US ».

Lorsque dans le présent texte est utilisée l'expression liste CMR UE, on entend liste CMR UE1 et/ou CMR UE2.

Les solvants utilisés dans le cadre de la présente invention sont ainsi dépourvus des familles de solvants et solvants suivants :

- certains alcanes, tels que l'hexane, l'heptane,...
- certains hydrocarbures aromatiques, tels que le naphtalène,

- certains solvants halogénés, notamment les solvants chlorés (1,2-dichloroéthane ou DCE, trichloroéthane, dichlorométhane, trichlorométhane (chloroforme), dichloroéthylène, tétrachlorure de carbone,...), ou encore le 1-chlorobutane.

Un des objectifs plus particulier est d'obtenir une fraction insaponifiable spécifique, par exemple ayant une teneur plus forte en certains composés, ou fractions, et éventuellement moins forte en d'autres, en particulier comprenant uniquement une ou certaines des familles de composés de l'insaponifiable total.

Les insaponifiables peuvent être composés de nombreux constituants, en particulier comprenant les grandes familles de substances définies ci-dessus et/ou des familles spécifiques. Il peut être souhaitable d'extraire le plus complètement possible au moins une de ces familles, notamment au moins deux, en particulier au moins trois, tout particulièrement au moins quatre, voire au moins cinq, tout particulièrement au moins six, et encore plus particulièrement toutes les familles composant l'insaponifiable d'une huile ou d'un beurre donné.

Le procédé selon l'invention peut ainsi tout particulièrement viser à améliorer le rendement global en insaponifiable et/ou le taux d'extraction en une ou plusieurs fractions, notamment deux, trois ou quatre fractions.

Autrement exprimé, le procédé selon l'invention peut viser soit à permettre l'obtention d'une fraction partielle spécifique de l'insaponifiable, notamment présentant une teneur enrichie en au moins une des familles composant l'insaponifiable, voire d'extraire spécifiquement un ou plusieurs composés particulier de l'insaponifiable, soit à permettre d'obtenir la fraction insaponifiable dite totale.

La présente invention a ainsi pour objet un procédé d'extraction d'une fraction insaponifiable, notamment partielle ou totale, contenue dans une huile ou un beurre végétal, dans une huile provenant d'un micro-organisme, dans un concentrât d'huile ou de beurre végétal ou d'huile provenant d'un micro-organisme, ou dans un co-produit de l'industrie du raffinage des huiles végétales, tel que les échappées de désodorisation et les distillats de raffinage physique, ou des huiles provenant de micro-organismes, comprenant au moins :

A) une étape de transformation desdites huiles, dudit beurre ou dudit co-produit de l'industrie du raffinage des huiles végétales, tel que les échappées de désodorisation et les distillats de raffinage, ou des huiles provenant de micro-organismes en solution hydro-alcoolique, notamment via une étape de saponification,
A') une étape d'ajustement du titre en alcool de la solution hydro-alcoolique obtenue à l'issue de l'étape A), et
B) une étape d'extraction de la solution hydro-alcoolique dans laquelle la fraction grasse est séparée de la fraction insaponifiable par une extraction liquide / liquide, et
C) éventuellement, une étape de purification de l'insaponifiable,

ledit procédé étant caractérisé en ce qu'au moins l'étape d'extraction liquide/liquide de l'étape B est effectuée en utilisant un premier système de solvants comprenant une teneur en solvant choisi parmi les solvants comprenant au moins 5 atomes de carbone, avantageusement entre 5 et 9 atomes de carbone, en particulier de 5 à 8 atomes de carbone, et un ou deux atomes d'oxygène, sous forme soit de fonction éther, soit de fonction cétone, soit de fonction ester, d'au moins 50% en volume par rapport au volume total du premier système de solvants,
lesdits solvants étant choisis parmi les méthylcétones, notamment la méthyl isobutyl cétone (MIBK), la 2-heptanone, les propionates, notamment l'éthyl propionate, le n-butyl propionate, l'isoamyl propionate, les propyléthers, notamment le diisopropyl éther (DIPE), et leurs mélanges, caractérisé en ce que, suite à l'étape A, l'étape A' d'ajustement du titre en alcool de la solution hydro-alcoolique permet d'obtenir un titre en alcool compris entre 10 et 50% en masse.

Par « système de solvants », on entend au sens de la présente invention un seul solvant ou un mélange de solvants.

Par « micro-organisme » on entend tout organisme vivant microscopique tel que les bactéries ou des champignons, notamment des levures et des moisissures.

Avantageusement, l'étape de saponification est réalisée en présence d'un excès de base de type potasse de manière à assurer une conversion totale des triglycérides en savons.

L'étape d'extraction liquide / liquide comprend typiquement une phase continue et une phase dispersée.

Par « phase continue » on entend la phase (organique ou aqueuse), remplissant la colonne d'extraction ; par « phase dispersée » on entend la phase (organique ou aqueuse) qui est présente sous forme de gouttelettes dans la colonne d'extraction.

La demanderesse a découvert de manière surprenante que plusieurs caractéristiques techniques, avantageusement combinées entre elles, permettaient d'optimiser l'extraction liquide / liquide et en particulier de jouer sur le rendement et sur la sélectivité de l'extraction (obtention d'un insaponifiable enrichi en telle ou telle fraction).

Il s'agit en particulier :

- de l'ajustement du titre en alcool de la solution hydro-alcoolique obtenue à l'issue de l'étape A ;
- de la nature de la phase continue lors de l'étape d'extraction B ; du ratio des débits de la solution hydro-alcoolique (ou phase aqueuse) et du solvant (ou phase organique), ou encore du ratio des volumes de solution hydro-alcoolique et de solvant mis en contact ;de la nature de l'insaponifiable considéré.

Dans un mode de réalisation particulier, le procédé selon la présente invention comprend une étape C de purification, suite à l'étape B d'extraction, ladite étape C de purification étant avantageusement choisie dans le groupe constitué par les cristallisations, les distillations, les rectifications, les précipitations, les filtrations, notamment les nano et ultrafiltrations, les désodorisations, les chromatographies liquides et les extractions liquide / liquide.

L'étape C de purification est alors typiquement effectuée en utilisant le premier système de solvants tel que défini ci-dessus, comprenant une teneur en solvant choisi parmi les solvants comprenant au moins 5 atomes de carbone, avantageusement entre 5 et 9 atomes de carbone, en particulier entre 5 et 8 atomes de carbone, et un ou deux atomes d'oxygène, sous forme soit de fonction éther, soit de fonction cétone, soit de fonction ester, d'au moins 50% en volume par rapport au volume total du premier système de solvants.

De manière particulièrement avantageuse, le procédé selon l'invention comprend une étape A' d'ajustement du titre en alcool de la solution hydro-alcoolique obtenue à l'issue de l'étape A, en particulier en vue d'optimiser l'étape suivante d'extraction B.

Dans un mode de réalisation particulier, on ajoute à la solution hydro-alcoolique un mélange eau / alcool pouvant comprendre de 0 à 40% d'alcool, plus particulièrement de 0 à 20% d'alcool et typiquement de 0 à 16% d'alcool.

L'alcool utilisé peut-être choisi parmi l'éthanol, le propanol, le n-butanol ou l'isopropanol.
La masse de mélange eau / alcool ajoutée pour ajuster le titre en alcool est de 1 à 5 fois la masse de solution hydro-alcoolique obtenue après saponification, plus particulièrement de 1 à 4 fois la masse de solution hydro-alcoolique obtenue après saponification, et typiquement de 1 à 2,2 fois la masse de solution hydro-alcoolique obtenue après saponification.

Lorsque le premier système de solvant comprend une teneur en un solvant ou un mélange de solvants choisis dans une liste de X %, cela signifie que le pourcentage complémentaire correspond à un ou plusieurs solvants organique(s), ne figurant pas dans cette liste.

Selon un mode de réalisation particulier, le premier système de solvant est dépourvu de tertbutyl éthers, en particulier de ETBE et /ou de MTBE ou encore de terpènes, en particulier de limonène et d'alpha-pinène..

En particulier, ledit solvant comprenant au moins 5 atomes de carbone et un ou deux atomes d'oxygène est choisi parmi les méthylcétones, notamment la méthyl isobutyl cétone ou MIBK et la 2-heptanone, les propionates, notamment l'éthyl propionate, le n-butyl propionate et l'isoamyl propionate, les propyléthers, notamment le diisopropyl éther ou DIPE, et leurs mélanges.

Les numéros CAS de ces différents solvants sont les suivants, méthyl isobutyl cétone ou MIBK: 108-10-1 ; 2-heptanone: 110-43-0 ; éthyl propionate: 105-37-3 ; butyl propionate: 590-01-2 ; isoamyl propionate : 105-68-0 ; diisopropyl éther ou DIPE : 108-20-3.

De manière particulièrement avantageuse, le solvant d'extraction est choisi parmi les méthylcétones, notamment la méthyl isobutyl cétone ou MIBK et la 2-heptanone, les propionates, notamment l'éthyl propionate, le n-butyl propionate et l'isoamyl propionate, et leurs mélanges.

Selon une caractéristique particulière de la présente invention, ledit solvant est une méthylcétone, telle que la méthyl isobutyl cétone ou MIBK.

Selon une autre caractéristique particulière de la présente invention, ledit solvant est le 2-heptanone.

Selon une autre caractéristique particulière de la présente invention, ledit solvant est au moins un propionate, notamment l'éthyl propionate, le n-butyl propionate et l'isoamyl propionate,

Selon une autre caractéristique particulière de la présente invention, le DIPE est utilisé en mélange avec un autre solvant d'extraction choisi parmi les méthylcétones, notamment la méthyl isobutyl cétone ou MIBK et la 2-heptanone, les propionates, notamment l'éthyl propionate, le n-butyl propionate et l'isoamyl propionate.

De manière particulièrement avantageuse, les solvants d'extraction liquide / liquide selon l'invention comprennent entre 5 et 8 atomes de carbone, et présentent ainsi de petites chaînes carbonées, et donc l'avantage d'être suffisamment lipophiles pour extraire notamment les insaponifiables de l'huile et de pouvoir être ensuite facilement séparés des insaponifiables suite à leur extraction.

Par « fraction totale », on entend au sens de la présente invention le fait que cette fraction comprend toutes les familles de substances composant l'insaponifiable présentes dans l'huile végétale ou dans l'huile provenant d'un micro-organisme ou dans le beurre végétal considéré.

Par « fraction partielle », on entend au sens de la présente invention le fait que cette fraction comprend au moins une des familles de substances composant l'insaponifiable présentes dans l'huile ou le beurre végétal ou l'huile provenant d'un micro-organisme considéré.

Tout particulièrement avantageusement, l'invention concerne un procédé dans lequel l'étape B) comprend, ou consiste en, une extraction liquide / liquide avec le premier système de solvants.

Selon un mode de réalisation particulier, l'invention concerne un procédé dans lequel l'étape C) comprend, ou consiste en, une cristallisation, une précipitation ou une chromatographie liquide avec le premier système de solvants.

Selon un mode de réalisation encore plus particulier, le procédé comprend une étape B) comprenant, ou consistant en, une extraction liquide / liquide avec un premier système de solvants et une étape C) comprenant, ou consistant en une cristallisation, une précipitation ou une chromatographie liquide avec un premier système de solvants identique ou différent de celui utilisé en étape B).

Tout particulièrement, l'étape C) peut permettre de purifier la fraction insaponifiable, d'enrichir celle-ci en une ou plusieurs familles de substances composant l'insaponifiable présente(s) dans l'huile végétale, l'huile provenant d'un micro-organisme ou le beurre végétal considéré.

En particulier, cela peut permettre d'isoler une fraction spécifique de l'insaponifiable de soja, tels que les composés stéroliques, les tocophérols et/ou le squalène ou de l'insaponifiable d'avocat, tels que les composés furaniques, trihydroxylés et/ou stéroliques.

Le procédé selon l'invention, notamment dans ses étapes A), B) et C, peut être dépourvu d'une étape de complexation impliquant le premier système de solvant.

Le premier système de solvants peut comprendre une teneur en solvant choisi parmi les solvants comprenant au moins 5 atomes de carbone, voire de 5 à 8, et un ou deux atomes d'oxygène sous forme de fonction éther, cétone ou ester, en particulier choisi parmi les méthylcétones, notamment la MIBK, la 2-heptanone, les propionates, notamment l'éthyl propionate, le n-butyl propionate, l'isoamyl propionate, les propyléthers, notamment DIPE, et leurs mélanges, d'au moins 60 %, notamment au moins 75%, en particulier au moins 90%, plus particulièrement au moins 95%, encore plus particulièrement au moins 99% en volume, par rapport au volume total du premier système de solvants.

En particulier, le premier système de solvants est constitué de solvant(s) comprenant au moins 5 atomes de carbone, voire de 5 à 8 atomes de carbone, et un ou deux atomes d'oxygène sous forme de fonction éther, cétone ou ester, en particulier choisi(s) parmi les méthylcétones, notamment la MIBK, la 2-heptanone, les propionates, notamment l'éthyl propionate, le n-butyl propionate, l'isoamyl propionate, les propyléthers, notamment le DIPE, ou un mélange de ceux-ci.

Le premier système de solvants peut comprendre une teneur en un seul solvant choisi parmi les solvants comprenant au moins 5 atomes de carbone, voire de 5 à 8 atomes de carbone, et un ou deux atomes d'oxygène sous forme de fonction éther, cétone ou ester, en particulier choisi parmi les méthylcétones, notamment la MIBK, la 2-heptanone, les propionates, notamment l'éthyl propionate, le n-butyl propionate, l'isoamyl propionate, , d'au moins 60%, notamment au moins 75%, en particulier au moins 90%, plus particulièrement au moins 95%, encore plus particulièrement au moins 99% en volume par rapport au volume total du premier système de solvants.

Selon une variante, le premier système de solvants est constitué par un solvant comprenant au moins 5 atomes de carbone, voire de 5 à 8 atomes de carbone, et un ou deux atomes d'oxygène sous forme de fonction éther, cétone ou ester, en particulier choisi parmi les méthylcétones, notamment la MIBK, la 2-heptanone, les propionates, notamment l'éthyl propionate, le n-butyl propionate, l'isoamyl propionate..

Selon un mode de réalisation particulier, le premier système de solvant présente une densité inférieure à 1, et notamment inférieure ou égale à 0,9.

Plus particulièrement, le premier système de solvants présente un écart de densité avec la solution hydro-alcoolique diluée (SHD) supérieur à 0,1 et plus particulièrement supérieur à 0,13, et spécifiquement supérieur à 0,2.

Avantageusement selon la présente invention, le premier système de solvants comprend en outre de l'hexaméthyldisiloxane (HMDS), typiquement à une teneur comprise entre 0,1 et 49% en volume, par rapport au volume total du premier système de solvant
Les numéros CAS du HMDS est le 107-46-0.

De manière avantageuse, l'étape B d'extraction liquide/liquide, ainsi qu'éventuellement l'étape C de purification, est (sont) effectuée(s) en utilisant un tel premier système de solvant comprenant notamment de l'HMDS.

Selon un mode de réalisation particulier, le premier système de solvant peut comprendre :

- une teneur en solvant choisi parmi les solvants comprenant au moins 5 atomes de carbone, en particulier de 5 à 8 atomes de carbone , et un ou deux atomes d'oxygène sous forme soit de fonction éther, soit de fonction cétone, soit de fonction ester, en particulier choisi parmi les méthylcétones, notamment la MIBK, la 2-heptanone, les propionates, notamment l'éthyl propionate, le n-butyl propionate, l'isoamyl propionate, les propyléthers, notamment le DIPE, et leurs mélanges, d'au moins 50% en volume par rapport au volume total du premier système de solvants, et
- du HMDS, notamment en une teneur allant de 0,1 à 49%, tout particulièrement de 0,5 à 30%, voire de 1 à 20%, et tout particulièrement de 5 à 10 % en volume, par rapport au volume total du premier système de solvant.

Le premier système de solvants peut comprendre :

- une teneur en solvant choisi parmi les solvants comprenant au moins 5 atomes de carbone, voire de 5 à 8 atomes de carbone, et un ou deux atomes d'oxygène sous forme de fonction éther, cétone ou ester, en particulier choisi parmi les méthylcétones, notamment la MIBK, la 2-heptanone, les propionates, notamment l'éthyl propionate, le n-butyl propionate, l'isoamyl propionate, les propyléthers, notamment le DIPE, et leurs mélanges, d'au moins 60%, notamment au moins 75%, en particulier au moins 90%, et plus particulièrement au moins 95% en volume par rapport au volume total du premier système de solvants, et

- du HMDS, notamment en une teneur allant de 0,1 à 40%, tout particulièrement de 0,5 à 25%, voire de 1 à 10%, et tout particulièrement de 5 à 10% en volume par rapport au volume total du premier système de solvant.

En particulier, le premier système de solvants est constitué :

- de solvant comprenant au moins 5 atomes de carbone, voire de 5 à 8 atomes de carbone, et un ou deux atomes d'oxygène sous forme de fonction éther, cétone ou ester, en particulier choisi parmi les méthylcétones, notamment la MIBK, la 2-heptanone, les propionates, notamment l'éthyl propionate, le n-butyl propionate, l'isoamyl propionate, les propyléthers, notamment le DIPE, ou d'un mélange de ceux-ci, avantageusement à une teneur d'au moins 50% en volume, par rapport au volume total du premier système de solvant, et
- de HMDS, notamment en une teneur allant de 0,1 à 49%, tout particulièrement de 0,5 à 30%, voire de 1 à 20%, et tout particulièrement de 5 à 10 % en volume par rapport au volume total du premier système de solvant.

Le premier système de solvants peut comprendre :

- une teneur en un solvant choisi parmi les solvants comprenant au moins 5 atomes de carbone, voire de 5 à 8 atomes de carbone, et un ou deux atomes d'oxygène sous forme de fonction éther, cétone ou ester, en particulier choisi parmi les méthylcétones, notamment la MIBK, la 2-heptanone, les propionates, notamment l'éthyl propionate, le n-butyl propionate, l'isoamyl propionate, les propyléthers, notamment le DIPE, d'au moins 60%, notamment au moins 75%, en particulier au moins 90%, plus particulièrement au moins 95%, encore plus particulièrement au moins 99% en volume par rapport au volume total du premier système de solvants, et
- du HMDS, notamment en une teneur allant de 0,1 à 40%, tout particulièrement de 0,5 à 25%, voire de 1 à 10%, et tout particulièrement de 5 à 10% en volume par rapport au volume total du premier système de solvant.

Selon une variante, le premier système de solvants est constitué par :

- un solvant comprenant au moins, 5 atomes de carbone, voire de 5 à 8 atomes de carbone, et un ou deux atomes d'oxygène sous forme de fonction éther, cétone ou ester, en particulier choisi parmi les méthylcétones, notamment la MIBK, la 2-heptanone, les propionates, notamment l'éthyl propionate, le n-butyl propionate, l'isoamyl propionate, les propyléthers, notamment le DIPE, avantageusement à une teneur d'au moins 50% en volume, par rapport au volume total du premier système de solvant, et
- du HMDS, notamment en une teneur allant de 0,1 à 49%, tout particulièrement de 0,5 à 25%, voire de 1 à 10%, et tout particulièrement de 5 à 10% en volume par rapport au volume total du premier système de solvant.

Selon un mode de réalisation particulier, le premier système de solvants comprend une teneur en solvant(s) CMR, en particulier présent(s) sur la liste CMR UE1, UE2, et/ou US, inférieure ou égale à 10%, notamment inférieure ou égale à 5%, en particulier inférieure ou égale à 2%, tout particulièrement inférieure ou égale à 1%, encore plus particulièrement inférieure ou égale à 0,5%, voire inférieure ou égale à 0,1% en volume, par rapport au volume total du premier système de solvants.

Encore plus particulièrement, le premier système de solvants est dépourvu de solvants présents sur la liste CMR UE1, UE2 et/ou US.

Les solvants utilisés dans le premier système de solvants présentent une pureté d'au moins 90%, notamment d'au moins 95%, en particulier d'au moins 98%, tout particulièrement d'au moins 99%, voire d'au moins 99,5%.

En particulier, l'étape A) de transformation d'huile, de beurre ou de co-produit de l'industrie du raffinage des huiles végétales ou de micro-organismes en solution hydro-alcoolique, notamment via une étape de saponification, est effectuée dans un deuxième système de solvants comprenant une teneur en solvant choisi parmi les alcools de C2 à C4, et notamment l'éthanol, le n-propanol, l'iso-propanol, le butanol, en particulier le n-butanol, le MeTHF et leurs mélanges d'au moins 50% en volume, par rapport au volume total du deuxième système de solvants.

Le deuxième système de solvants peut comprendre une teneur en solvant choisi parmi les alcools de C2 à C4, et notamment l'éthanol, le n-propanol, l'iso-propanol, le butanol, en particulier le n-butanol, le MeTHF et leurs mélanges d'au moins 60 %, notamment au moins 75%, en particulier au moins 90%, plus particulièrement au moins 95%, encore plus particulièrement au moins 99% en volume, par rapport au volume total du deuxième système de solvants.

En particulier, le deuxième système de solvants est constitué d'éthanol, de n-propanol, d'iso-propanol, de butanol, de MeTHF ou d'un mélange de ceux-ci.

Le deuxième système de solvants peut comprendre une teneur en un solvant choisi parmi les alcools de C2 à C4, et notamment l'éthanol, le n-propanol, l'iso-propanol, le butanol, en particulier le n-butanol, et le MeTHF, d'au moins 50 %, notamment au moins 75%, en particulier au moins 90%, plus particulièrement au moins 95%, encore plus particulièrement au moins 99% en volume par rapport au volume total du deuxième système de solvants.

Selon un mode de réalisation particulier, le deuxième système de solvants comprend une teneur en solvant(s) présent(s) sur la liste CMR UE1, UE2 et/ou US, inférieure ou égale à 10%, notamment inférieure ou égale à 5%, en particulier inférieure ou égale à 2%, tout particulièrement inférieure ou égale à 1%, encore plus particulièrement inférieure ou égale à 0,5%, voire inférieure ou égale à 0,1% en volume par rapport au volume total du deuxième système de solvants.

Encore plus particulièrement le deuxième système de solvants est dépourvu de solvants présents sur la liste CMR UE1, UE2 et/ou US.

Les solvants utilisés dans le deuxième système de solvants présentent une pureté d'au moins 90%, notamment d'au moins 95%, en particulier d'au moins 98%, tout particulièrement d'au moins 99%, voire d'au moins 99,5%.

Par « solution hydroalcoolique diluée (SHD) », on entend au sens de la présente invention le milieu réactionnel de saponification dilué dans l'eau avantageusement déminéralisée et comprenant notamment de l'eau et un ou plusieurs solvants très polaires, en particulier choisis parmi les alcools, par exemple en C2 à C4, et le MeTHF.

La solution hydroalcoolique diluée (SHD) peut comprendre une teneur en eau supérieure ou égale à 50%, notamment supérieure ou égale à 60%, en particulier supérieure ou égale à 65%, tout particulièrement supérieure ou égale à 70%, voire supérieure ou égale à 72% en volume par rapport au volume de la solution hydroalcoolique.

La solution hydroalcoolique diluée (SHD) peut comprendre une teneur en eau inférieure ou égale à 95%, notamment inférieure ou égale à 90%, en particulier inférieure ou égale à 85%, tout particulièrement inférieure ou égale à 80%, voire inférieure ou égale à 75% en volume par rapport au volume de la solution hydroalcoolique.

Tout particulièrement, la SHD peut comprendre un titre en alcool compris entre 10 et 50%, plus particulièrement entre 11 et 40%, et typiquement entre 12 et 28 %, en masse, le titre en alcool étant fonction du solvant considéré et de la nature de l'insaponifiable.

Plus particulièrement, lors de l'extraction d'insaponifiable d'avocat, la SHD peut comprendre un titre en alcool compris entre 10 et 20%, typiquement entre 10 et 15%.

Lors de l'extraction d'insaponifiable de soja, la SHD peut comprendre un titre en alcool compris entre 20 et 30% et typiquement entre 22% et 28%.

Le titre en alcool est ajusté pour optimiser l'extraction, notamment en termes de partage des phases entre la SHD et le premier de système de solvant. L'ajustement du titre en alcool, avantageusement couplé à l'ajustement du ratio des volumes ou débits de SHD et solvant mis en contact et de la nature de la phase continue, permet d'améliorer l'efficacité, la sélectivité et/ou la qualité de l'opération d'extraction B. L'optimisation de ces paramètres a pour effet d'améliorer la productivité du procédé notamment en maximisant le rendement d'extraction de l'étape B, en diminuant la quantité de solvant à mettre enjeu, en réduisant les temps de contact entre les phases, en diminuant les consommations énergétiques, en facilitant le déphasage et/ou en diminuant les risques d'émulsions, et en diminuant la quantité de déchets générés. Elle permet également d'améliorer la sélectivité de l'opération d'extraction en favorisant l'extraction d'une, de deux, de trois, de quatre, voire de toutes les fractions de l'insaponifiable et/ou la qualité de l'extraction en limitant l'extraction de composés connexes, notamment en optimisant la séparation des phases et limitant la part d'eau entrainée dans la phase organique.

La présente invention a encore pour objet un procédé d'extraction d'une fraction insaponifiable, notamment partielle ou totale, contenue dans une huile végétale, une huile provenant d'un micro-organisme, un beurre végétal ou un co-produit de l'industrie du raffinage des huiles végétales, notamment d'avocat et/ou de soja, ou des huiles provenant de micro-organismes comprenant au moins:

A) une étape de saponification par laquelle ladite huile, ledit beurre ou ledit co-produit de l'industrie du raffinage des huiles végétales ou des huiles provenant de micro-organimes est transformé après saponification en une solution hydro-alcoolique,

B) une étape d'extraction de la solution hydro-alcoolique par un premier système de solvants tel que défini ci-dessus.

Plus particulièrement, le procédé d'extraction de la fraction insaponifiable selon la présente invention est tel que l'on effectue une extraction liquide / liquide en mettant en contact la SHD avec un premier système de solvants, notamment à contre-courant, dans lequel le rapport (volume/volume) système de solvants / SHD va de 0,1 à 10, avantageusement de 0,1 à 5, typiquement de 0,2 à 5, notamment de 0,25 à 5, en particulier de 0,5 à 2 ou de 0,2 à 1,2, voire 0,4 à 1,2.

Plus particulièrement, le procédé d'extraction de la fraction insaponifiable est tel que l'on peut effectuer une extraction liquide / liquide en mettant en contact la SHD avec un premier système de solvants, notamment à contre-courant, au moyen d'un premier système de solvants tel que défini ci-dessus et comprenant, voire consistant en, de la MIBK, et éventuellement du HMDS, avantageusement le rapport (volume/volume) système de solvants / SHD allant de 0,1 à 10, notamment de 0,25 à 5, en particulier de 0,5 à 2.

Plus particulièrement, le procédé d'extraction de la fraction insaponifiable est tel que l'on effectue une extraction liquide / liquide en mettant en contact la SHD avec un premier système de solvants, notamment à contre-courant, au moyen d'un premier système de solvants tel que défini ci-dessus et comprenant, voire consistant en, du DIPE, et éventuellement du HMDS, avantageusement le rapport (volume/volume) système de solvants / SHD allant de 0,1 à 10, notamment de 0,25 à 5, en particulier de 0,5 à 2.

Plus particulièrement, le procédé d'extraction de la fraction insaponifiable est tel que l'on effectue une extraction liquide / liquide en mettant en contact la SHD avec un premier système de solvants, notamment à contre-courant, au moyen d'un premier système de solvants tel que défini ci-dessus et comprenant, voire consistant en, de l'éthyl propionate, et éventuellement du HMDS, avantageusement le rapport (volume/volume) système de solvants / SHD allant de 0,1 à 10, notamment de 0,25 à 5, en particulier de 0,5 à 2.

Plus particulièrement, la nature de la phase continue pourra être soit la SHD à extraire, soit le solvant d'extraction. La nature de la phase continue est définie en fonction du titre en alcool de la SHD préparée, du ratio des volumes ou débits de SHD et de solvant mis en contact et de l'insaponifiable considéré.

L'huile végétale ou l'huile provenant de micro-organisme utilisée dans le présent procédé peut être choisie parmi l'huile de soja, de quinoa, de colza, de maïs, de tournesol, de sésame, de lupin, de coton, de noix de coco, d'olive, de palme, de germe de blé, de luzerne, d'avocat, de palmiste, d'arachide, de coprah, de lin, de ricin, de pépins de raisin, de pépins de courge, de pépins de cassis, de pépins de melon, de pépins de tomate, de pépins de citrouille, d'amande, de noisette, de noix, d'onagre, de bourrache, de carthame, de cameline, d'oeillette, de macro-algues, de micro-algues, comme Chorella, et/ou provenir de micro-organismes, notamment marin, d'eau douce ou terrestre, en particulier de levures, de moisissures, et plus particulièrement, de bactéries, et leurs mélanges.

Le beurre végétal peut être choisi parmi le beurre de cacao, d'illipé, de karité, et leurs mélanges.

La comparaison des teneurs en insaponifiables de différentes huiles végétales : soja, coton, noix de coco, olive et avocat, montre que l'huile d'avocat obtenue par extraction suivant divers procédés connus comprend un taux particulièrement important d'insaponifiable.

Typiquement, les teneurs de fraction insaponifiable obtenues s'échelonnent de 2 à 10% dans l'huile d'avocat, sont d'environ 0,5% dans l'huile de coco, d'environ 1% dans l'huile de soja et d'environ 1 % dans l'huile d'olive.

L'insaponifiable d'avocat peut être préparé par extraction à partir d'huile d'avocat.

Le procédé d'extraction de l'insaponifiable d'une huile d'avocat peut être effectué de la façon suivante.

Selon une méthode connue de l'homme du métier pour extraire l'huile :

- soit on presse la pulpe fraîche en présence d'un tiers corps fibreux absorbant l'eau, tel que la parche de café, dans une presse à cage, puis on sépare l'émulsion d'huile et d'eau obtenue par décantation et/ou centrifugation;
- soit on broie la pulpe fraîche et on la met en contact avec un solvant organique adapté (par exemple un mélange méthanol-chloroforme) puis on récupère l'huile par évaporation du solvant.

Plusieurs procédés ont été décrits dans l'art antérieur pour extraire la fraction insaponifiable d'une huile végétale.

On peut citer en particulier le procédé de préparation d'insaponifiable d'huile d'avocat tel que décrit et revendiqué dans le brevet FR 2 678 632.

Ainsi, l'insaponifiable d'huile d'avocat utilisé selon l'invention peut être obtenu à partir du fruit frais mais, de préférence, insaponifiable d'avocat est préparé à partir du fruit préalablement traité thermiquement, avant l'extraction de l'huile et la saponification, comme décrit dans le brevet FR 2 678 632.

Ce traitement thermique consiste en un séchage contrôlé du fruit, frais de préférence, pendant au moins quatre heures, avantageusement au moins 10 heures, de préférence entre environ 24 et environ 48 heures, à une température de préférence d'au moins environ 80°C et de préférence comprise entre environ 80 et environ 120°C.

On peut également citer le procédé de préparation d'insaponifiable d'huile de soja, obtenu à partir d'un concentrât d'insaponifiable d'huile de soja.

Ledit concentrât d'insaponifiable est préparé par distillation moléculaire selon un procédé tel que décrit pour l'huile de lupin dans la demande de brevet FR 2 762 512, mais adapté à l'huile de soja.

Dans ce procédé, l'huile de soja est distillée dans un distillateur moléculaire de type centrifuge ou à film raclé, à une température comprise entre environ 210 et 250°C et sous un vide poussé, compris entre 0,01 et 0,001 millimètres de mercure (soit 0,13 à 1,3 Pa).

Le distillat obtenu présente une teneur en insaponifiable comprise entre 5 et 40% en volume et constitue donc un concentrât d'insaponifiable d'huile de soja.

Le concentrât est ensuite saponifié par une base telle que la potasse ou la soude en milieu polaire, notamment alcoolique, de préférence de l'éthanol, du n-propanol, de l'iso-propanol, du butanol, en particulier le du n-butanol, du MeTHF, ou un mélange de ceux-ci, puis il est soumis à une ou plusieurs extractions par le premier système de solvants.

La solution d'extraction obtenue est de préférence ensuite centrifugée, filtrée puis lavée à l'eau pour éliminer les traces résiduelles d'alcalinité.

Le solvant d'extraction est évaporé soigneusement pour récupérer l'insaponifiable.

Enfin, avant sa saponification, l'huile peut être préalablement enrichie en insaponifiable en séparant une majorité des constituants de l'insaponifiable que l'on récupère dans un concentrât. Différentes méthodes peuvent être utilisées : cristallisation par le froid, extraction liquide / liquide, ou encore distillation moléculaire.

La concentration préalable de l'huile en insaponifiable permet de diminuer les volumes d'huile à saponifier.

La distillation moléculaire est particulièrement préférée, étant réalisée de préférence à une température comprise entre environ 180 et environ 230°C en maintenant une pression comprise entre $10^{-3}$ et $10^{-2}$ mm Hg et de préférence de l'ordre de $10^{-3}$ mm Hg.

La concentration en insaponifiable du distillat peut atteindre 60% en masse par rapport à la masse totale.

Tout particulièrement, la présente invention concerne un procédé tel que décrit ci-dessus dans lequel l'insaponifiable obtenu est choisi parmi un insaponifiable de soja, un insaponifiable d'avocat, notamment un insaponifiable d'avocat riche en fraction furanique et/ou un insaponifiable d'avocat riche en fraction stérolique.

Le procédé selon la présente invention permet d'extraire une fraction insaponifiable contenue dans une huile végétale, une huile provenant d'un micro-organisme ou un beurre végétal, il peut également permettre d'extraire une fraction insaponifiable à partir d'un co-produit de l'industrie du raffinage des huiles végétales ou des huiles provenant d'un micro-organisme, comme par exemple les échappées de désodorisation, encore appelées déodistillats, produites au cours du raffinage des huiles végétales ou des huiles provenant de micro-organismes.

Les acides gras et les glycérides partiels présents dans les déodistillats peuvent en effet être saponifiés ou estérifiés par un alcool léger, dans le but de séparer la fraction grasse de la fraction insaponifiable, soit par extraction liquide / liquide soit par distillation sous vide.

Enfin, la purification de l'insaponifiable ou des fractions actives séparées, le plus souvent les tocophérols (incluant la vitamine E) et les stérols, met en jeu notamment des étapes de cristallisation dans un solvant organique ou d'extraction liquide / liquide.

La présente invention a également pour objet un procédé d'extraction de la fraction insaponifiable dans un co-produit de l'industrie du raffinage d'une huile végétale ou d'une huile provenant d'un micro-organisme, tel que ce co-produit est un déodistillat d'une huile végétale ou d'une huile provenant d'un micro-organisme, ledit procédé comprenant au moins :

- une étape de saponification transformant le déodistillat en une solution hydro-alcoolique,
- une étape d'extraction à contre-courant de la solution hydro-alcoolique au moyen du premier système de solvants,
- une étape de cristallisation des stérols et/ou des alcools triterpéniques,
- une étape de séparation d'un composé actif, tel que les tocophérols, les tocotriénols, le squalène et les carotènes, ladite étape de séparation étant choisie dans le groupe formé par les extractions, en particulier au moyen du premier système de solvants, et les distillations.

Tout particulièrement, la cristallisation des stérols et/ou des alcools triterpéniques peut être effectuée dans le premier système de solvants.

La présente invention a encore pour objet une fraction insaponifiable, notamment partielle ou totale, dépourvue de solvants classés dans la liste CMR UE1, UE2 et/ou US, susceptible d'être obtenue, par le procédé d'extraction selon la présente invention, en particulier ladite fraction est directement obtenue par le procédé d'extraction selon la présente invention.

La présente description concerne encore l'utilisation de cette fraction pour la préparation d'une composition, notamment pharmaceutique, alimentaire et/ou cosmétique, ou encore d'un complément alimentaire.

La présente description a également pour objet la fraction insaponifiable, notamment partielle ou totale, dépourvue de solvants classés dans la liste CMR UE1, UE2 et/ou US, telle que décrite ci-dessus, pour son utilisation comme médicament, comme dispositif médical, comme agent dermatologique, comme agent cosmétique, ou comme nutraceutique, à visée humaine ou animale, avantageusement dans la prévention et/ou le traitement des troubles du tissu conjonctif tels que l'arthrose, des pathologies articulaires telles que les rhumatismes, des maladies parodontales, telles que la gingivite ou la parodontite, ou encore dans la prévention et/ou le traitement des troubles du derme et/ou de l'hypoderme tels que le vieillissement cutané, les vergetures et la cellulite, ou encore des troubles de la barrière épidermique tels que les inflammations cutanées, l'eczéma atopique et les dermatites irritatives et/ou inflammatoires.

La présente description a encore pour objet une composition, notamment alimentaire, cosmétique ou pharmaceutique, ou encore un complément alimentaire, comprenant au moins une fraction insaponifiable d'au moins une huile végétale ou d'une huile provenant d'un micro-organisme, ladite fraction étant dépourvue de solvants classés dans la liste CMR UE1, UE2 et/ou US et/ou ladite fraction étant susceptible d'être obtenue, ou directement obtenue, par le procédé selon l'invention, et ladite composition comprenant éventuellement un excipient, en particulier cosmétiquement, alimentairement ou pharmaceutiquement acceptable.

Selon un mode de réalisation particulier, la présente description concerne une composition, notamment pharmaceutique, alimentaire ou cosmétique, ou encore un complément alimentaire, comprenant au moins un insaponifiable, en particulier un insaponifiable de soja, un insaponifiable d'avocat, tout particulièrement un insaponifiable d'avocat riche en fraction furanique et/ou un insaponifiable d'avocat riche en fraction stérolique, susceptible d'être obtenu ou directement obtenu par le procédé selon l'invention.

Les compositions peuvent être destinées à la prévention et/ou au traitement de troubles du tissu conjonctif, notamment de l'arthrose, des parodonthopathies, du vieillissement cutané et/ou des inflammations cutanées.

Les compositions cosmétiques peuvent être destinées à la prévention et/ou au traitement des troubles cutanées de l'épiderme du derme et/ou de l'hypoderme.

Par « dépourvu de solvants classés dans la liste CMR UE1, UE2 et/ou US », on entend au sens de la présente invention une teneur totale en solvants classés dans la liste CMR UE1, UE2 et/ou US inférieure à 10 ppm, notamment inférieure à 5 ppm, en particulier inférieure à 2 ppm, voire inférieure à 1 ppm.

La présente description vise encore un procédé de traitement cosmétique tel que l'on applique de façon topique la composition cosmétique selon l'invention.

La description concerne aussi un insaponifiable d'une huile végétale, d'une huile provenant d'un micro-organisme ou d'un beurre végétal obtenu ou susceptible d'être obtenu selon le procédé de la présente invention pour son utilisation en tant que médicament, en particulier destiné à traiter ou à prévenir des troubles du tissu conjonctif, et notamment l'arthrose.

Selon encore un autre aspect, la description a pour objet l'utilisation de HMDS, en une teneur allant de 0,1 à 49%, typiquement de 0,1 à 45%, en volume, par rapport au volume total des solvants d'extraction, dans un procédé d'extraction liquide/liquide d'insaponifiable, avantageusement à partir d'une solution hydro-alcoolique.

En particulier, le HMDS est présent en une teneur allant de 0,5 à 25%, voire de 1 à 10%, et tout particulièrement de 5 à 10% en volume, par rapport au volume total de solvant.

En particulier ledit procédé selon l'invention comprend un système de solvant comprenant :

- au moins un solvant comprenant au moins 5 atomes de carbone, voire de 5 à 8 atomes de carbone, et un ou deux atomes d'oxygène sous forme de fonction éther, cétone ou ester, en particulier au moins un solvant choisi parmi

  ◦ les méthylcétones, notamment la MIBK, la2-heptanone,
  ◦ les propionates, notamment l'éthyl propionate, le n-butyl propionate, l'isoamyl propionate,
  ◦ les propyléthers, notamment le DIPE, et
  ◦ un mélange de ceux-ci, ou

- au moins un solvant choisi parmi

  ◦ les solvants aromatiques fluorés, notamment le trifluorotoluène (BTF) et l'hexafluorobenzène (BHF),
  ◦ les tert-butyl éthers, notamment le 2-éthoxy-2-méthylpropane, encore appelé éthyl-tert-butyl-éther (ETBE),
  ◦ les solvants comprenant au moins un atome de silicium, notamment l'hexaméthyldisiloxane (HMDS) et le tétraméthylsilane (TMS),
  ◦ le méthyl-tétrahydrofurane (MeTHF), et
  ◦ leurs mélanges, ou

- un mélange de solvants ci-dessus.

La teneur en ces solvants peut être telle que décrite ci-dessus.

Les numéros CAS de ces différents solvants sont les suivants BTF : 98-08-8 ; BHF : 392-56-3 ; ETBE : 637-92-3 ; TMS : 75-76-3 ; et MeTHF : 96-47-9.

Le HMDS peut notamment permettre de moduler la capacité extractive du système de solvant. Ainsi, la présence de HMDS parmi les solvants d'extraction peut permettre d'affiner le profil de l'insaponifiable obtenu, d'améliorer le rendement global d'extraction et/ou le taux d'extraction en une ou plusieurs fractions.

Par ailleurs, la présence de HMDS dans une teneur telle que définie ci-dessus dans un système de solvants peut permettre de diminuer la consommation de solvants, d'eau de lavage et/ou de temps d'extraction. Cela peut encore permettre de faciliter la décantation et ainsi provoquer une formation d'émulsion moindre et/ou un déphasage plus rapide au cours des étapes d'extraction et/ou de lavage.

Ainsi, le HMDS peut être utilisé en tant qu'agent d'amélioration du rendement et/ou du taux d'extraction, agent modulateur des profils d'insaponifiables, agent déphasant et/ou agent d'accélération.

Bien entendu, les différentes caractéristiques exposées dans la présente description peuvent être combinées entre elles.

A titre d'exemples illustrant la présente invention, les expérimentations suivantes ont été effectuées.

**EXEMPLES**

Exemple 1 : Extraction d'insaponifiable d'avocat avec du DCE (référence 1)

La première étape consiste à saponifier un concentrât préparé par distillation moléculaire d'huile d'avocat.

A cet effet, dans un ballon de 100 ml équipé d'un réfrigérant sont successivement introduits une masse donnée de matière grasse d'avocat (15,6 g) puis de l'éthanol (36,6 mL), de la potasse à 50% (5,2 mL) et quelques grains de pierre ponce.

Le système est ensuite porté à reflux pendant 3h30 puis, après refroidissement, dilué avec de l'eau déminéralisée ce qui permet de modifier le titre en alcool de la solution saponifiée à 21% massique.

La solution hydro-alcoolique diluée obtenue contient l'insaponifiable (ou fraction d'insaponifiable) en solution. Cet insaponifiable est alors extrait par un premier système de solvants, en l'occurrence le DCE.

Plusieurs extractions successives (5 x 60 mL) sont réalisées ; les phases organiques ainsi collectées sont ensuite rassemblées et lavées à l'eau (5 x 150 mL) jusqu'à neutralité.

La phase solvant obtenue est alors séchée sur sulfate de sodium anhydre puis filtrée ; l'insaponifiable est ensuite récupéré par évaporation du solvant à l'évaporateur rotatif en mettant en oeuvre une température de 60°C et une pression de l'ordre de 300mbar ; il est ensuite séché sous vide poussé.

L'insaponifiable ainsi extrait est pesé et stocké dans un pilulier sous atmosphère inerte. Les résultats sont présentés dans le tableau 1 ci-après.

Exemple 2 : Extraction d'insaponifiable d'avocat avec de le 2-heptanone

L'extraction est effectuée selon le mode opératoire de l'Exemple 1 qui, après optimisation pour l'adapter au nouveau système de solvants, correspond à :

- une étape de dilution permettant d'ajuster le titre en alcool à 21% massique ;
- une étape d'extraction avec 5 x 60 ml de 2-heptanone;
- une étape de lavage des phases organiques avec 6 x 150mL d'eau.

Les mesures sont effectuées comme dans l'Exemple 1 et les résultats sont présentés dans le tableau 1 ci-après.

Exemple 3 : Extraction d'insaponifiable d'avocat avec de l'isoamyl propionate

L'extraction est effectuée selon le mode opératoire de l'Exemple 1 qui, après optimisation pour l'adapter au nouveau système de solvants, correspond à :

- une étape de dilution permettant d'ajuster le titre en alcool à 22% massique ;
- une étape d'extraction avec 5 x 60 ml d'isoamyl propionate;
- une étape de lavage des phases organiques avec 6 x 150mL d'eau.

Les mesures sont effectuées comme dans l'Exemple 1 et les résultats sont présentés dans le tableau 1 ci-après.

Exemple 4 : Extraction d'insaponifiable d'avocat avec du n-butyl propionate

L'extraction est effectuée selon le mode opératoire de l'Exemple 1 à ceci près que les quantités de matière mise enjeu sont divisées par 2.

Après optimisation pour l'adapter au nouveau système de solvants, le mode opératoire utilisé correspond à :

- une étape de dilution permettant d'ajuster le titre en alcool à 22% massique ;
- une étape d'extraction avec 5 x 30 mL d'isoamyl propionate;
- une étape de lavage des phases organiques avec 6 x 75mL d'eau.

Les mesures sont effectuées comme dans l'Exemple 1 et les résultats sont présentés dans le tableau 1 ci-après.

Tableau 1

| Delta de rendement entre le procédé de référence (extraction au DCE - Exemple 1) et les procédés décrits dans les exemples 2 à 4 | | |
|---|---|---|
| | Rendement massique en insaponifiable (%) | Gain de rendement (%) |
| Exemple 1 | 38,7 | 0 |
| Exemple 2 | 47,6 | +23 |
| Exemple 3 | 48,4 | +25 |
| Exemple 4 | 51,7 | +34 |

Le rendement massique en insaponifiable, est calculé de la façon suivante :

$$R = 100 \text{ x (Masse d'insaponifiable extrait / Masse de concentrât mis en jeu)}$$

Le gain en rendement est calculé de la façon suivante :

$$G = 100 \text{ x [(R lié au solvant S – R lié au solvant de référence) / R lié au solvant de référence ]}$$

Les résultats montrent bien que le rendement massique en insaponifiable des procédés selon l'invention est amélioré par rapport au procédé impliquant du DCE.

Exemple 5 : Extraction d'insaponifiable de soja avec du DCE (référence 2)

La première étape consiste à saponifier un déodistillat de soja.
A cet effet, dans un ballon de 100 ml équipé d'un réfrigérant sont successivement introduits une masse donnée de désodistillat de soja (10 g) puis de l'éthanol (23,3 mL), de la potasse à 50% (1,7 mL) et quelques grains de pierre ponce.
Le système est ensuite porté à reflux pendant 3h00 puis, après refroidissement, dilué avec de l'eau déminéralisée ce qui permet de modifier le titre en alcool de la solution saponifiée à 23% massique.
La solution hydro-alcoolique diluée obtenue contient l'insaponifiable (ou fraction d'insaponifiable) en solution. Cet insaponifiable est alors extrait par un premier système de solvants, en l'occurrence le DCE.
Plusieurs extractions successives (5 x 36 mL) sont réalisées ; les phases organiques ainsi collectées sont ensuite rassemblées et lavées à l'eau de ville (5 x 90 mL) jusqu'à neutralité.
La phase solvant obtenue est alors séchée sur sulfate de sodium anhydre puis filtrée ; l'insaponifiable est ensuite récupéré par évaporation du solvant à l'évaporateur rotatif en mettant en oeuvre une température de 60°C et une pression de l'ordre de 300mbar ; il est ensuite séché sous vide poussé.
L'insaponifiable ainsi extrait est pesé et stocké dans un pilulier sous atmosphère inerte. Les résultats sont présentés dans le tableau 2 ci-après.

Exemple 6 : Extraction d'insaponifiable de soja avec de la MIBK

L'extraction est effectuée selon le mode opératoire de l'Exemple 5 qui, après optimisation pour l'adapter au nouveau système de solvants, correspond à :

-   une étape de dilution permettant d'ajuster le titre en alcool à 23% massique ;
-   une étape d'extraction avec 2 x 105 mL de MIBK;
-   une étape de lavage des phases organiques avec 6 x 105mL d'eau.

Les mesures sont effectuées comme dans l'Exemple 5 et les résultats sont présentés dans le tableau 2 ci-après

Exemple 7 : Extraction d'insaponifiable de soja avec de l'éthyl propionate

L'extraction est effectuée selon le mode opératoire de l'Exemple 5 qui, après optimisation pour l'adapter au nouveau système de solvants, correspond à :

- une étape de dilution permettant d'ajuster le titre en alcool à 23% massique ;
- une étape d'extraction avec 5 x 60 mL d'ethyl proprionate;
- une étape de lavage des phases organiques avec 6 x 150 mL d'eau.

Les mesures sont effectuées comme dans l'Exemple 5 et les résultats sont présentés dans le tableau 2 ci-après

Exemple 8 : Extraction d'insaponifiable de soja avec un mélange de MIBK et de HMDS 90/10 v/v

L'extraction est effectuée selon le mode opératoire de l'Exemple 5 qui, après optimisation pour l'adapter au nouveau système de solvants, correspond à :

- une étape de dilution permettant d'ajuster le titre en alcool à 24% massique ;
- une étape d'extraction avec 2 x 105 mL d'un mélange MIBK / HMDS 90/10 v/v;
- une étape de lavage des phases organiques avec 6 x 105 mL d'eau.

Les mesures sont effectuées comme dans l'Exemple 5 et les résultats sont présentés dans le tableau 2 ci-après

Tableau 2

| Delta de rendement entre le procédé de référence (extraction au DCE - Exemple 5) et les procédés décrits dans les exemples 6 à 8 | | |
|---|---|---|
| | Rendement massique en insaponifiable (%) | Gain de rendement (%) |
| Exemple 5 | 46,2 | 0 |
| Exemple 6 | 50,4 | +9 |
| Exemple 7 | 50,0 | +8 |
| Exemple 8 | 45,0 | -3 |

Le rendement massique en insaponifiable, est calculé de la façon suivante :

$$R = 100 \text{ x (Masse d'insaponifiable extrait / Masse de concentrât mis en jeu)}$$

Le gain en rendement est calculé de la façon suivante :

$$G = 100 \text{ x [(R lié au solvant S – R lié au solvant de référence) / R lié au solvant de référence ]}$$

Les exemples 6 et 7, montrent bien que le rendement massique en insaponifiable des procédés selon l'invention est amélioré par rapport au procédé impliquant du DCE.
Par ailleurs, l'exemple 8, en restant dans une gamme de rendement massique en insaponifiable voisine de la cible, permet de gagner en qualité de déphasage et donc en temps global d'extraction.

Exemple 9 : Extraction d'insaponifiable d'avocat à contre-courant avec du DCE

L'essai présenté dans l'exemple 1 a été transposé à l'échelle pilote en réalisant une extraction à contre-courant dans une colonne agitée. Le titre de la solution hydroalcoolique a été ajusté à 24% après la saponification via une étape de dilution. Au démarrage de l'extraction, la colonne est remplie de solvant (phase continue) et le ratio des débits solvant ou phase organique Vs SHD ou solution hydroalcoolique diluée est fixé à 1,2.
Le rendement massique de cette extraction a été calculé et est de 42%.

Exemple 10 : Extraction d'insaponifiable de soja à contre-courant avec du DCE

L'essai présenté dans l'exemple 5 a été transposé à l'échelle pilote en réalisant une extraction à contre-courant dans une colonne agitée. Le titre de la solution hydroalcoolique a été ajusté à 23% après la saponification via une étape de dilution. Au démarrage de l'extraction, la colonne est remplie de solvant (phase continue) et le ratio des débits solvant ou phase organique Vs SHD ou solution hydroalcoolique diluée est fixé à 1,2.

Le rendement massique de cette extraction a été calculé et est de 40%.

Exemple 11 : Extraction d'insaponifiable d'avocat à contre-courant avec de la MIBK

L'essai présenté dans l'exemple 6 a été transposé à l'échelle pilote sur une matrice avocat en réalisant une extraction à contre-courant dans une colonne agitée. Le titre de la solution hydroalcoolique a été ajusté à 14% après la saponification via une étape de dilution. Au démarrage de l'extraction, la colonne est remplie de solvant (phase continue) et le ratio des débits solvant ou phase organique Vs SHD ou solution hydroalcoolique est fixé à 0,9.

Le rendement massique de cette extraction a été calculé et est de 50%.

Exemple 12 : Extraction d'insaponifiable d'avocat à contre-courant avec de la MIBK

L'essai présenté dans l'exemple 6 a été transposé à l'échelle pilote sur une matrice avocat en réalisant une extraction à contre-courant dans une colonne agitée. Le titre de la solution hydroalcoolique a été ajusté à 14% après la saponification via une étape de dilution. Au démarrage de l'extraction, la colonne est remplie de solution hydroalcoolique (phase continue) et le ratio des débits solvant ou phase organique Vs SHD ou solution hydroalcoolique est fixé à 0,38. Le rendement massique de cette extraction a été calculé et est de 45%.

Exemple 13 : Extraction d'insaponifiables de soja à contre-courant avec de la MIBK

L'essai présenté dans l'exemple 6 a été transposé à l'échelle pilote en réalisant une extraction à contre-courant dans une colonne agitée. Le titre de la solution hydroalcoolique a été ajusté à 24% après la saponification via une étape de dilution. Au démarrage de l'extraction, la colonne est remplie de solvant (phase continue) et le ratio des débits solvant ou phase organique Vs SHD ou solution hydroalcoolique est fixé à 1,2. Le rendement massique de cette extraction a été calculé et est de 39%.

Le tableau ci-dessous synthétise les résultats obtenus :

|  | Soja | | Avocat | | |
|---|---|---|---|---|---|
| Solvant | DCE | MIBK | DCE | MIBK | MIBK |
| Exemple N° | 10 | 13 | 9 | 11 | 12 |
| Phase continue | Solvant | Solvant | Solvant | Solvant | Aqueuse |
| Titre en alcool | 23% | 24% | 24% | 14% | 14% |
| Ratio des débits | 1,2 | 1,2 | 1,2 | 0,90 | 0,38 |
| Rendement | 40% | 39% | 42% | 50% | 45% |

Les essais menés montrent que le choix du titre en alcool de la SHD influe sur le partage des phases et permet d'optimiser le rendement d'extraction.

**Revendications**

1.  Procédé d'extraction d'une fraction insaponifiable contenue dans une huile ou un beurre végétal, dans une huile provenant d'un micro-organisme, dans un concentrât d'huile ou de beurre végétal ou d'huile provenant d'un micro-organisme, ou dans un co-produit de l'industrie du raffinage des huiles végétales, tel que les échappées de désodorisation et les distillats de raffinage physique, ou des huiles provenant de micro-organismes, comprenant au moins :

    A) une étape de transformation desdites huiles, dudit beurre ou dudit co-produit de l'industrie du raffinage des huiles végétales ou des huiles provenant de micro-organismes en solution hydro-alcoolique, notamment via une étape de saponification,
    A') une étape d'ajustement du titre en alcool de la solution hydro-alcoolique obtenue à l'issue de l'étape A), et
    B) une étape d'extraction de la solution hydro-alcoolique dans laquelle la fraction grasse est séparée de la fraction insaponifiable par une extraction liquide / liquide,

ledit procédé étant **caractérisé en ce qu'**au moins l'étape d'extraction liquide / liquide de l'étape B est effectuée en utilisant un premier système de solvants comprenant une teneur en solvant choisi parmi les solvants comprenant au moins 5 atomes de carbone et un ou deux atomes d'oxygène sous forme soit de fonction éther, soit de fonction cétone, soit de fonction ester, d'au moins 50% en volume par rapport au volume total du premier système de solvants, lesdits solvants étant choisis parmi les méthylcétones, notamment la méthyl isobutyl cétone (MIBK), la 2-heptanone, les propionates, notamment l'éthyl propionate, le n-butyl propionate, l'isoamyl propionate, les propyléthers, notamment le diisopropyl éther (DIPE), et leurs mélanges, **caractérisé en ce que**, suite à l'étape A, l'étape A' d'ajustement du titre en alcool de la solution hydro-alcoolique permet d'obtenir un titre en alcool compris entre 10 et 50% en masse.

2. Procédé selon la revendication 1, **caractérisé en ce que**, suite à l'étape A, l'étape A' d'ajustement du titre en alcool de la solution hydro-alcoolique permet d'obtenir un titre en alcool compris entre 11 et 40% en masse et typiquement entre 12 et 28% en masse

3. Procédé selon la revendication 1 ou 2, **caractérisé en ce que**, suite à l'étape B, il comprend en outre une étape C de purification de l'insaponifiable.

4. Procédé selon la revendication 3, **caractérisé en ce que** l'étape C de purification de l'insaponifiable est effectuée en utilisant ledit premier système de solvants.

5. Procédé selon l'une quelconque des revendications 1 à 4, **caractérisé en ce que** le premier système de solvant présente une densité inférieure à 1.

6. Procédé selon l'une quelconque des revendications précédentes, **caractérisé en ce que** le premier système de solvant comprend une teneur en solvant comprenant au moins 5 atomes de carbone et un ou deux atomes d'oxygène sous forme de fonction éther, cétone ou ester d'au moins 60%, notamment au moins 75%, en particulier au moins 90%, plus particulièrement au moins 95%, encore plus particulièrement au moins 99% en volume, par rapport au volume total du premier système de solvants.

7. Procédé selon l'une quelconque des revendications précédentes, **caractérisé en ce que** le premier système de solvants comprend une teneur en un seul solvant choisi parmi les méthylcétones, notamment la méthyl isobutyl cétone (MIBK), la 2-heptanone, les propionates, notamment l'éthyl propionate, le n-butyl propionate, l'isoamyl propionate, d'au moins 60%, notamment au moins 75%, en particulier au moins 90%, plus particulièrement au moins 95%, encore plus particulièrement au moins 99% en volume, par rapport au volume total du premier système de solvants.

8. Procédé selon l'une quelconque des revendications précédentes, **caractérisé en ce que** le premier système de solvant comprend en outre du hexaméthyldisiloxane (HMDS), avantageusement en une teneur allant de 0,1 à 49 % en volume par rapport au volume total du premier système de solvant.

9. Procédé selon l'une quelconque des revendications précédentes, **caractérisé en ce que** le premier système de solvant comprend une teneur en solvant(s) CMR, en particulier présent(s) sur la liste CMR UE1, UE2, et/ou US, inférieure ou égale à 10%, notamment inférieure ou égale à 5%, en particulier inférieure ou égale à 2%, tout particulièrement inférieure ou égale à 1%, encore plus particulièrement inférieure ou égale à 0,5%, voire inférieure ou égale à 0,1% en volume, par rapport au volume total du premier système de solvants.

10. Procédé selon l'une quelconque des revendications précédentes, **caractérisé en ce que** l'étape A) de transformation d'huile, de beurre ou de co-produit de l'industrie du raffinage des huiles végétales ou d'huile provenant de micro-organismes en solution hydro-alcoolique est effectuée dans un deuxième système de solvants comprenant une teneur en solvant choisi parmi les alcools de C2 à C4, et notamment l'éthanol, le n-propanol, l'iso-propanol, le butanol, en particulier le n-butanol, le méthyl-tétrahydrofurane (MeTHF) et leurs mélanges, d'au moins 50% en volume par rapport au volume total du deuxième système de solvants.

11. Procédé selon la revendication 10, **caractérisé en ce que** le deuxième système de solvants comprend une teneur en solvant choisi parmi les alcools de C2 à C4, et notamment l'éthanol, le n-propanol, l'iso-propanol, le butanol, en particulier le n-butanol, le méthyl-tétrahydrofurane (MeTHF) et leurs mélanges, d'au moins 60%, notamment au moins 75%, en particulier au moins 90%, plus particulièrement au moins 95%, encore plus particulièrement au moins 99% en volume, par rapport au volume total du deuxième système de solvants.

**12.** Procédé selon la revendication 10 ou 11, **caractérisé en ce que** le deuxième système de solvants comprend une teneur en solvant(s) présent(s) sur la liste CMR UE1, UE2 et/ou US, inférieure ou égale à 10%, notamment inférieure ou égale à 5%, en particulier inférieure ou égale à 2%, tout particulièrement inférieure ou égale à 1%, encore plus particulièrement inférieure ou égale à 0,5%, voire inférieure ou égale à 0,1% en volume, par rapport au volume total du deuxième système de solvants.

**13.** Procédé selon l'une quelconque des revendications précédentes, **caractérisé en ce que** le titre en alcool de la solution hydro-alcoolique est compris entre 10 et 50 % en masse.

**14.** Procédé selon l'une quelconque des revendications précédentes, **caractérisé en ce que** :

- l'huile végétale ou l'huile provenant de micro-organisme utilisée dans le présent procédé est choisie parmi l'huile de soja, de quinoa, de colza, de maïs, de tournesol, de sésame, de lupin, de coton, de noix de coco, d'olive, de palme, de germe de blé, de luzerne, d'avocat, de palmiste, d'arachide, de coprah, de lin, de ricin, de pépins de raisin, de pépins de courge, de pépins de cassis, de pépins de melon, de pépins de tomate, de pépins de citrouille, d'amande, de noisette, de noix, d'onagre, de bourrache, de carthame, de cameline, d'oeillette, de macro-algues, de micro-algues, comme Chorella, et/ou provenir de micro-organismes, notamment marin, d'eau douce ou terrestre, en particulier de levures, de moisissures, et plus particulièrement, de bactéries, et leurs mélanges, et
- le beurre végétal est choisi parmi le beurre de cacao, d'illipé, de karité, et leurs mélanges.

**15.** Procédé selon l'une quelconque des revendications précédentes, **caractérisé en ce que** l'insaponifiable obtenu est choisi parmi un insaponifiable de soja, un insaponifiable d'avocat, notamment un insaponifiable d'avocat riche en fraction furanique et/ou un insaponifiable d'avocat riche en fraction stérolique.

**Patentansprüche**

**1.** Verfahren zur Extraktion einer unverseifbaren Fraktion, die in einem pflanzlichen Öl oder Butter enthalten ist, in einem Öl aus einem Mikroorganismus in einem Konzentrat von pflanzlichen Öl oder Butter oder Öl aus einem Mikroorganismus oder in einem Nebenprodukt der Raffinerieindustrie für pflanzliche Öle wie z. B. Abfallprodukten der Desodorierung und den Destillaten der physischen Raffinierung oder Ölen aus Mikroorganismen, umfassend mindestens:

A) einen Schritt des Umwandelns der Öle, der Butter oder des Nebenprodukts der Raffinerieindustrie für pflanzliche Öle oder Öle aus Mikroorganismen in Wasser-Alkohol-Lösung, vor allem mit Hilfe eines Schritts des Verseifens,
A') einen Schritt des Einstellens der Alkoholstärke der Wasser-Alkohol-Lösung, die am Ausgang von Schritt A) erhalten wurde, und
B) einen Schritt des Extrahierens der Wasser-Alkohol-Lösung, in dem die fette Fraktion von der unverseifbaren Fraktion durch Flüssig-Flüssig-Extraktion getrennt wird,

wobei das Verfahren **dadurch gekennzeichnet ist, dass** mindestens der Schritt des Flüssig-Flüssig-Extrahierens von Schritt B unter Verwendung eines ersten Lösemittelsystems durchgeführt wird, umfassend einen Gehalt an Lösemitteln, ausgewählt aus den Lösemitteln, umfassend mindestens 5 Kohlenstoffatome und ein oder zwei Sauerstoffatome entweder als Etherfunktion, als Ketonfunktion oder als Esterfunktion von mindestens 50 Volumenprozent mit Bezug auf das Gesamtvolumen des ersten Lösungsmittelsystems, wobei die Lösemittel ausgewählt sind aus Methylketonen, vor allem Methylisobutylketon (MIBK), 2-Heptanon, Propionaten, vor allem Ethylpropionat, n-Butylpropionat, Isoamylpropionat, Propylethern, vor allem Diisopropylether (DIPE) und ihren Mischungen, **dadurch gekennzeichnet, dass**, nach Schritt A der Schritt A' des Einstellens der Alkoholstärke der Wasser-Alkohol-Lösung ermöglicht, eine Alkoholstärke im Bereich zwischen 10 und 50 Massenprozent zu erhalten.

**2.** Verfahren nach Anspruch 1, **dadurch gekennzeichnet, dass** nach Schritt A der Schritt A' des Einstellens der Alkoholstärke der Wasser-Alkohol-Lösung ermöglicht, eine Alkoholstärke im Bereich zwischen 11 und 40 Massenprozent und typischerweise zwischen 12 und 28 Massenprozent zu erhalten.

**3.** Verfahren nach Anspruch 1 oder 2, **dadurch gekennzeichnet, dass** es nach Schritt B außerdem einen Schritt C des Reinigens des unverseifbaren Teils umfasst.

4.  Verfahren nach Anspruch 3, **dadurch gekennzeichnet, dass** der Schritt C des Reinigens des unverseifbaren Teils unter Verwendung des ersten Lösemittelsystems durchgeführt wird.

5.  Verfahren nach einem beliebigen der Ansprüche 1 bis 4, **dadurch gekennzeichnet, dass** das erste Lösemittelsystem eine Dichte von weniger als 1 aufweist.

6.  Verfahren nach einem der vorhergehenden Ansprüche, **dadurch gekennzeichnet, dass** das erste Lösemittelsystem einen Gehalt an Lösemitteln aufweist, der mindestens 5 Kohlenstoffatome und ein oder zwei Sauerstoffatome in Form einer Ether-, Keton- oder Esterfunktion von mindestens 60, vor allem mindestens 75, insbesondere mindestens 90, ganz besonders mindestens 95, noch bevorzugter mindestens 99 Volumenprozent mit Bezug auf das Gesamtvolumen des ersten Lösemittelsystems umfasst.

7.  Verfahren nach einem der vorhergehenden Ansprüche, **dadurch gekennzeichnet, dass** das erste Lösemittelsystem einen Gehalt eines einzigen Lösemittels umfasst, ausgewählt aus Methylketonen, vor allem Methylisobutylketon (MIBK), 2-Heptanon, Propionaten, vor allem Ethylpropionat, n-Butylpropionat, Isoamylpropionat von mindestens 60, vor allem mindestens 75, insbesondere mindestens 90, ganz besonders mindestens 95, noch bevorzugter mindestens 99 Volumenprozent mit Bezug auf das Gesamtvolumen des ersten Lösemittelsystems umfasst.

8.  Verfahren nach einem der vorhergehenden Ansprüche, **dadurch gekennzeichnet, dass** das erste Lösemittelsystem außerdem Hexamethyldisiloxan (HMDS) umfasst, vorzugsweise mit einem Gehalt von 0,1 bis 49 Volumenprozent mit Bezug auf das Gesamtvolumen des ersten Lösemittelsystems umfasst.

9.  Verfahren nach einem der vorhergehenden Ansprüche, **dadurch gekennzeichnet, dass** das erste Lösemittelsystem einen Gehalt von Lösemittel(n) CMR, insbesondere vorhanden auf der Liste CMR UE1, UE2 und/oder US, von weniger als oder gleich wie 10, vor allem weniger als oder gleich wie 5, insbesondere weniger als oder gleich wie 2, ganz besonders weniger als oder gleich wie 1, noch bevorzugter weniger als oder gleich wie 0,5, sogar weniger als oder gleich wie 0,1 Volumenprozent mit Bezug auf das Gesamtvolumen des ersten Lösemittelsystems umfasst.

10.  Verfahren nach einem der vorhergehenden Ansprüche, **dadurch gekennzeichnet, dass** der Schritt A) des Umwandelns von Öl, Butter oder Nebenprodukt der Raffinerieindustrie für pflanzliche Öle oder Öl aus Mikroorganismen in Wasser-Alkohol-Lösung in einem zweiten Lösemittelsystem durchgeführt wird, umfassend einen Gehalt an Lösemittel, ausgewählt aus Alkoholen von C2 bis C4 und vor allem Ethanol, n-Propanol, Isopropanol, Butanol, insbesondere n-Butanol, Methyltetrahydrofuran (MeTHF) und ihren Mischungen, von mindestens 50 Volumenprozent mit Bezug auf das Gesamtvolumen des zweiten Lösemittelsystems.

11.  Verfahren nach Anspruch 10, **dadurch gekennzeichnet, dass** das zweite Lösemittelsystem einen Gehalt an Lösemittel aufweist, ausgewählt aus den Alkoholen von C2 bis C4 und vor allem Ethanol, n-Propanol, Isopropanol, Butanol, insbesondere n-Butanol, Methyltetrahydrofuran (MeTHF) und ihren Mischungen, von mindestens 60, vor allem mindestens 75, insbesondere mindestens 90, ganz besonders mindestens 95, noch bevorzugter mindestens 99 Volumenprozent mit Bezug auf das Gesamtvolumen des zweiten Lösemittelsystems umfasst.

12.  Verfahren nach Anspruch 10 oder 11, **dadurch gekennzeichnet, dass** das zweite Lösemittelsystem einen Gehalt an Lösemittel, vorhanden in der Liste CMR UE1, UE2 und/oder US, von weniger als oder gleich wie 10, vor allem weniger als oder gleich wie 5, insbesondere weniger als oder gleich wie 2, ganz besonders weniger als oder gleich wie 1, noch bevorzugter weniger als oder gleich wie 0,5, sogar weniger als oder gleich wie 0,1 Volumenprozent mit Bezug auf das Gesamtvolumen des zweiten Lösemittelsystems umfasst.

13.  Verfahren nach einem der vorhergehenden Ansprüche, **dadurch gekennzeichnet, dass** die Alkoholstärke der Wasser-Alkohol-Lösung im Bereich zwischen 10 und 50 Massenprozent liegt.

14.  Verfahren nach einem der vorhergehenden Ansprüche, **dadurch gekennzeichnet, dass**

     - das pflanzliche Öl oder das Öl aus einem Mikroorganismus, das im vorliegenden Verfahren verwendet wird, ausgewählt ist aus Soja-, Quinoa-, Raps-, Mais-, Sonnenblumen-, Sesam-, Lupinen-, Baumwoll-, Kokos-, Oliven-, Palm-, Weizenkeim-, Luzernen-, Avocado-, Palmkern-, Erdnuss-, Kopra, Lein-, Rizinus-, Traubenkern-, Kürbiskern-, schwarzem Johannisbeeren-, Melonenkern-, Tomatenkern-, Gartenkürbiskern-, Mandel-, Haselnuss-, Walnuss-, Nachtkerzen-, Borretsch, Distel-, Leindotter-, Mohn-, Makroalgen-, Mikroalgen- wie z. B. Chlorella, und/oder aus Mikroorganismen, vor allem Meeres-, Süßwasser- oder Landorganismen, insbesondere Hefen, Schimmel und ganz besonders Bakterien und ihre Mischungen, und
- die pflanzliche Butter ausgewählt ist aus Kakao-, Illipe-, Karitebutter und ihre Mischungen.

**15.** Verfahren nach einem der vorhergehenden Ansprüche, **dadurch gekennzeichnet, dass** der erhaltene unverseifbare Teil ausgewählt ist aus unverseifbarem Soja, unverseifbarem Avocado, vor allem unverseifbarem Avocado reich an Furanfraktion und/oder unverseifbarem Avocado reich an Sterolfraktion.

## Claims

**1.** Method for extracting a non-saponifiable fraction contained in an oil or vegetable butter, in an oil from a microorganism, in a concentrate of oil or of vegetable butter or of oil from a microorganism, or in a co-product of the industry of refining vegetable oils, such as deodorisation distillates and physical-refining distillates, or oils from microorganisms, comprising at least:

A) a step of transforming said oils, said butter or said co-product of the industry of refining vegetable oils or oils from microorganisms into a hydroalcoholic solution, in particular via a saponification step,
A') a step of adjusting the alcohol titre of the hydroalcoholic solution obtained after step A), and
B) a step of extracting the hydroalcoholic solution in which the fatty fraction is separated from the non-saponifiable fraction by liquid/liquid extraction,

said method being **characterised in that** at least the step of liquid/liquid extraction of step B is carried out using a first system of solvents comprising a concentration of solvent, chosen from the solvents comprising at least 5 atoms of carbon and one or two atoms of oxygen in the form of an ether function, a ketone function or an ester function, of at least 50% by volume with respect to the total volume of the first system of solvents, said solvents being chosen from the methyl ketones, in particular methyl isobutyl ketone (MIBK), 2-heptanone, the propionates, in particular ethyl propionate, n-butyl propionate, isoamyl propionate, the propyl ethers, in particular diisopropyl ether (DIPE), and the mixtures thereof, **characterised in that** after step A, the step A' of adjusting the alcohol titre of the hydroalcoholic solution allows an alcohol titre between 10 and 50% by weight to be obtained.

**2.** Method according to claim 1, **characterised in that**, after step A, the step A' of adjusting the alcohol titre of the hydroalcoholic solution allows an alcohol titre between 11 and 40% by weight, and typically between 12 and 28% by weight, to be obtained.

**3.** Method according to claim 1 or 2, **characterised in that** after step B, it further comprises a step C of purifying the non-saponifiable material.

**4.** Method according to claim 3, **characterised in that** the step C of purifying the non-saponifiable material is carried out using said first system of solvents.

**5.** Method according to any of claims 1 to 4, **characterised in that** the first solvent system has a density of less than 1.

**6.** Method according to any of the previous claims, **characterised in that** the first solvent system comprises a concentration of solvent comprising at least 5 atoms of carbon and one or two atoms of oxygen in the form of an ether, ketone or ester function of at least 60%, namely at least 75%, in particular at least 90%, more particularly at least 95%, even more particularly at least 99% by volume, with respect to the total volume of the first system of solvents.

**7.** Method according to any of the previous claims, **characterised in that** the first system of solvents comprises a concentration of a single solvent chosen from the methyl ketones, in particular methyl isobutyl ketone (MIBK), 2-heptanone, the propionates, in particular ethyl propionate, n-butyl propionate, isoamyl propionate of at least 60%, namely at least 75%, in particular at least 90%, more particularly at least 95%, even more particularly at least 99% by volume, with respect to the total volume of the first system of solvents.

**8.** Method according to any of the previous claims, **characterised in that** the first solvent system further comprises hexamethyldisiloxane (HMDS), advantageously in a concentration from 0.1 to 49% by volume with respect to the total volume of the first solvent system.

9. Method according to any of the previous claims, **characterised in that** the first solvent system comprises a concentration of CMR solvent(s), in particular present on the list CMR UE1, UE2 and/or US, less than or equal to 10%, namely less than or equal to 5%, in particular less than or equal to 2%, particularly less than or equal to 1%, even more particularly less than or equal to 0.5%, or even less than or equal to 0.1% by volume with respect to the total volume of the first system of solvents.

10. Method according to any of the previous claims, **characterised in that** the step A) of transforming oil, butter or co-product of the industry of refining vegetable oils or oil from microorganisms into a hydroalcoholic solution is carried out in a second system of solvents comprising a concentration of solvent chosen from the C2 to C4 alcohols, and in particular ethanol, n-propanol, isopropanol, butanol, in particular n-butanol, methyltetrahydrofuran (MeTHF) and the mixtures thereof, of at least 50% by volume with respect to the total volume of the second system of solvents.

11. Method according to claim 10, **characterised in that** the second system of solvents comprises a concentration of solvent chosen from the C2 to C4 alcohols, and in particular ethanol, n-propanol, isopropanol, butanol, in particular n-butanol, methyltetrahydrofuran (MeTHF) and the mixtures thereof, of at least 60%, namely at least 75%, in particular at least 90%, more particularly at least 95%, even more particularly at least 99% by volume with respect to the total volume of the second system of solvents.

12. Method according to claim 10 or 11, **characterised in that** the second system of solvents comprises a concentration of solvent(s) present on the list CMR UE1, UE2 and/or US less than or equal to 10%, namely less than or equal to 5%, in particular less than or equal to 2%, particularly less than or equal to 1%, even more particularly less than or equal to 0.5%, or even less than or equal to 0.1% by volume with respect to the total volume of the second system of solvents.

13. Method according to any of the previous claims, **characterised in that** the alcohol titre of the hydroalcoholic solution is between 10 and 50% by weight.

14. Method according to any of the previous claims, **characterised in that**:

- the vegetable oil or the oil from a microorganism used in the present method is chosen from the oil of soybeans, quinoa, colza, corn, sunflower, sesame, lupine, cotton, coconut, olive, palm, wheat germ, alfalfa, avocado, palm kernel, peanut, copra, linseed, castor, grape seed, squash seed, blackcurrant seed, melon seed, tomato seed, pumpkin seed, almond, hazelnut, walnut, evening primrose, borage, safflower, false flax, poppy seed, macroalgae, microalgae, such as *Chorella*, and/or come from microorganisms, in particular marine, freshwater or terrestrial, in particular from yeast, from moulds, and more particularly, from bacteria, and the mixtures thereof, and
- the vegetable butter is chosen from cocoa, illipe, shea butter and the mixtures thereof.

15. Method according to any of the previous claims, **characterised in that** the non-saponifiable material obtained is chosen from a non-saponifiable of soybean, a non-saponifiable of avocado, in particular a non-saponifiable of avocado rich in furanic fraction and/or a non-saponifiable of avocado rich in sterolic fraction.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2530809 A
- CN 101318988
- FR 2951736
- US 3804819 A

- EP 1733731 A
- FR 2678632
- FR 2762512

**Littérature non-brevet citée dans la description**

- Chemicals known or suspected to cause cancer or reproductive toxicity. California department of public health, occupational health branch, California safe cosmetic program » liée à la « California Safe Cosmetics Act of 2005 ». 01 Septembre 2009